# EUROPEAN PATENT APPLICATION

(11) **EP 1 777 206 A1**
(43) Date of publication of application: **25.04.2007**
(21) Application number: 05077396.9
(22) Date of filing: 18.10.2005
(51) Int. Cl.: C04B 37/00, C04B 35/486, C04B 41/53, C04B 41/45, A61C 13/09, A61C 5/08, A61K 6/00

(54) **Surface treatment for zirconia based materials**

(71) Applicant: Universiteit van Amsterdam, 1012 WX Amsterdam (NL)
(72) Inventor: Aboushelib,Moustafa Nabil Moustafa, 1066 HZ Amsterdam (NL); Feilzer, Albert Joseph, 1081 KP Amsterdam (NL)
(74) Representative: Winckels, Johannes Hubertus F.

(57) **Abstract**

The invention is directed to a method by which the bonding strength between zirconia-based materials and other materials can be improved, and to zirconia-based materials obtainable by this method and which have improved surface properties.

According to the present invention there is provided a method for increasing the bond strength between stabilised zirconia and another material, wherein said stabilised zirconia comprises ZrO₂ and at least one stabilising compound, which method comprises the successive steps of:
i) applying a conditioning agent to said stabilised zirconia;
ii) heating said stabilised zirconia to a temperature above the glass transition temperature of said conditioning agent;
iii) cooling said stabilised zirconia; and
iv) washing said stabilised zirconia.

The method of the invention results in stabilised zirconia which has an improved bond strength with other materials, which bond strength is stable with time. The surface modified stabilised zirconia of the invention can be used in prosthetic restorations, implants, solid electrolytes, fuel cells, oxygen sensors, cutting abrasives, or electrical insulators.

## Description

The invention is directed to a method for improving the bonding strength between zirconia-based materials and other materials, and to zirconia-based materials obtainable by this method and which have improved surface properties.

Stabilised zirconia, and in particular zirconia stabilised with yttrium, is a material that is widely used in the preparation of ceramic compositions. This material is very promising and finds increasing use as replacement for metal alloys, such as in orthopaedic devices, dental prostheses and the like. Stabilised zirconia has amongst others excellent mechanical and chemical properties.

However, the inert outer surface of stabilised zirconia results in difficulties, because hardly any material can bond to it. Therefore, the bond strengths between zirconia and other materials, such as adhesive resins, ceramics, or bone structures, are in general relatively weak. In the case of implants, unsatisfactory bone integration with the surface of the implant is the result. Moreover, most known bonds between zirconia and other materials deteriorate with time.

Another problem using zirconia-based materials in *e.g.* dentistry is that the bond strength with veneering ceramics is weak. Veneering ceramics are often used on top of the zirconia-based material in order to obtain an aesthetically nice tooth. However, the weak bond strength between the zirconia-based materials and the veneering ceramics often results in delamination of the ceramic veneers (Dent Mater. 2005, 21, 984-991).

It has been proposed to improve the bonding strength of yttrium oxide partially stabilised zirconia (YPSZ) by using certain resin cements (J. Adhesion Dent. 2000, 2, 139-146). However, for clinical use with YPSZ only one resin cement (a chemically cured resin composite modified with phosphate monomer 10-methacryloyloxydecyl dihydrogenphosphate) was recommended.

Another well-known method for enhancing the bond strength to ceramic structures is by surface coating or conditioning. However, the homogeneous structure of zirconia is hard to acid etch and it was found that zirconia based materials do not respond successfully to common silica coating silanation procedures.

Object of the invention is therefore to provide a method by which stabilised zirconia material can bond with other materials with enhanced bonding strength.

A further object of the invention is to provide a stabilised zirconia material that does not require a specific resin cement or specific adhesive for bonding with other materials.

Another object of the invention is to provide a stabilised zirconia material that has long-term stable bond strength with other materials.

These and other objects have been met by the method of the invention for increasing the bond strength between stabilised zirconia and another material, wherein said stabilised zirconia comprises ZrO₂ and at least one stabilising compound, which method comprises the successive steps of:
- applying a conditioning agent to said stabilised zirconia;
- heating said stabilised zirconia to a temperature above the glass transition temperature of said conditioning agent;
- cooling said stabilised zirconia; and
- washing said stabilised zirconia.

The invention further provides a stabilised zirconia material which is a surface modified stabilised zirconia comprising ZrO₂ and at least one stabilising compound, and which is obtainable by the above-mentioned method.

It has been found that the stabilised zirconia according to the invention has an increased bond strength, which is stable with time.

Pure ZrO₂ has a monoclinic crystal structure at room temperature. Upon heating it may transition via a tetragonal crystal structures or to a cubic crystal structures at increasing temperatures. The volume expansion caused by the reversed transition (cubic → tetragonal → monoclinic transformation) upon cooling induces very large stresses, and will cause pure ZrO₂ to crack upon cooling from high temperatures. Several different oxides are added to zirconia to stabilise the tetragonal and/or cubic phases. Suitable stabilisers are for instance MgO, CaO, Y₂O₃, CeO₂, Al₂O₃, Sc₂O₃ and Yb₂O₃. Depending on the stabiliser used, partially stabilised zirconia (PSZ) or tetragonal zirconia polycrystals (TZP) may be obtained. The amount of stabiliser may vary from 0.05 to 10 mol%, more preferably between 0.1 and 5 mol%. Most preferred is yttrium stabilised TZP having 1-4 mol%, more preferably about 3 mol% of Y₂O₃.

In some cases, the tetragonal phase can be metastable. If sufficient quantities of the metastable tetragonal phase are present, then an applied stress, magnified by the stress concentration at a crack tip, can cause the tetragonal phase to convert to monoclinic, with the associated volume expansion. This phase transformation can put the crack into compression, retarding its growth, and enhancing the fracture toughness. This mechanism is known as transformation toughening, and significantly extends the reliability and lifetime of products made with stabilised zirconia.

A special case of zirconia is that of tetragonal zirconia polycrystalline or TZP, which is indicative of polycrystalline zirconia composed of only the metastable tetragonal phase. In TZP the use of extremely fine initial powders, and the application of low sintering temperatures, achieves an extremely fine-grained microstructure. Due to its extremely fine microstructure (grain size < 1-3 µm) and the metastable tetragonal structure, this material is characterised by extraordinary high mechanical strength, possibly even exceeding 1 500 MPa under flexure.

Because of its excellent properties tetragonal zirconia polycrystalline that is partially stabilised with yttrium oxide, is widely used. It should be noted that varieties may exist among these materials, even when these materials have the same chemical composition. Differences in e.g. strength and translucency may occur, for instance as a result of the chosen powder type and the production conditions. In dentistry, typically yttrium stabilised TZP is used with 2-3 mol% of Y₂O₃. This is generally referred to as 3Y-TZP.

Surprisingly, it has now been found that the bond strength of stabilised zirconia with other materials can be significantly improved by modifying the surface of the zirconia crystals, in a way that the surface becomes nanostructured. This results in increased surface energy and thus in an increased possibility to establish nano-retention with many materials. The stabilised zirconia according to the invention has excellent bond strength and can be used without the need for special surface, chemical, mechanical treatments or any specific bonding agents.

Typically, the zirconia according to the invention is prepared by the following procedure.

### Step I: Maturation Heat treatment

The maturation heat treatment is an optional pretreatment. This step is dependent on the chemical structure and physical structure of the used zirconia, especially the crystal sizes and the inter-grain structure. During the maturation heat treatment the stabilised zirconia crystals are first heated to a temperature above 800 °C. For most stabilised zirconia materials a temperature in the range of 800 - 1100 °C is suitable, preferably a range of 900 - 1000 °C is used. The zirconia crystals are preferably held at this temperature for a certain amount of time to allow maturation of the grain sizes. Typically, the hold time is 5 to 15 minutes, more preferably 8 to 12 minutes. Without wishing to be bound by theory it is believed that during the hold time of the heating step, the zirconia crystals at the surface undergo a monoclinic-tetragonal phase change. The selected heating temperature is therefore in the range of the tetragonal-monoclinic phase transformation temperature. Although this phase transformation temperature may vary with the type of stabilised zirconia used, it is typically above 800 °C. The phase transition is characterised by maturation of the grain structure of zirconia into more homogenous and evenly distributed shapes. Independent crystals at grain boundaries tend to join the neighbouring grains and thus create nano-spaces between the grains and result in a nano-structured surface on both the ultra-structured crystal level and on the micro-structural grain level. Thus, this heat treatment takes advantage of the dynamic transformation properties of zirconia and uses it to establish two basic structural changes:
A-Maturation of grain sizes, which is obtained through:
B-Redistribution of the zirconia crystals at the surface.

These two structural changes result in changing the bond strength between the zirconia crystals especially at the surface of the material.

Next, the crystals are cooled. This can for example be a cooling to ambient, but other ways of cooling may also be applied. It is believed that during this cooling the crystals at the surface undergo a reverse phase transition. These phase transitions have the effect that the bonds between the zirconia crystals at the surface weakens and that the average crystal size of the zirconia crystals, in some types of TZP, at the surface becomes smaller.

Although the effect on the zirconia surface is already significant after heating and cooling one time, in a preferred embodiment, the heating and cooling are switched several times, *e.g.* 2-10 times, in order to increase the change in crystal size and surface structure if deeper structural changes are required below the surface for a depth of more than 3-5 µm.

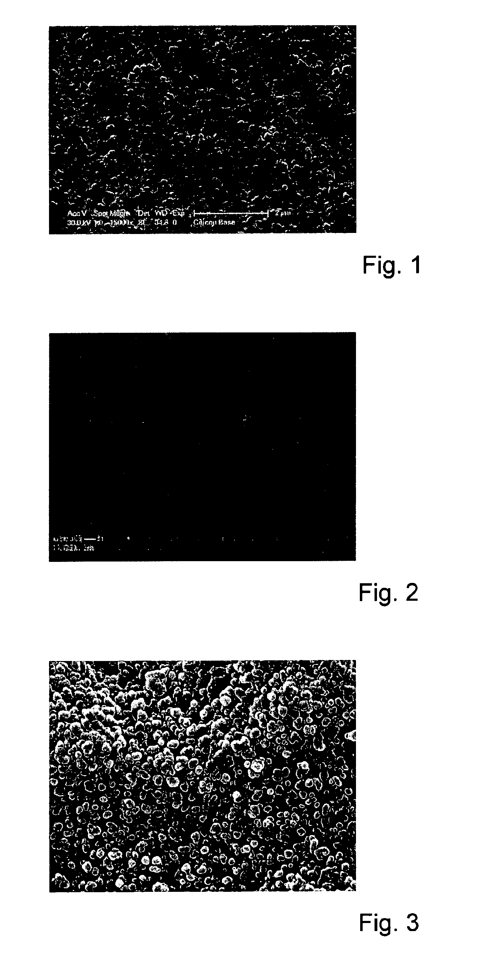

The change in surface properties upon the maturation heat treatment can be followed by scanning electron microscopy (SEM). Figures 1 and 2 show SEM images of a zirconia surface before and after subjecting the zirconia to maturation heat treatment.

### Step II: Selective Infiltration Etching

In many cases the maturation heat treatment step can be selectively added or combined to the selective infiltration etching step in order to shorten the preparation time.

In the selective infiltration etching step, a conditioning agent is applied, followed by a specific activation cycle, which allows the top layer zirconia crystals to be etched. The specific activation cycle comprises the application of a conditioning agent, heating above the glass transition temperature of the conditioning agent, cooling, and washing the remainder of conditioning agent. Adjusting the activation cycle allows a selective etching of zirconia crystals. Typical conditioning agents, which may be used, are acidulated or balanced non-ionised oxidised mixtures of basically aluminium, potassium, glass powder and silica. Trace elements, which help to reduce both time and temperature of this treatment, may be present as well. The conditioning agent is applied on the surface receiving treatment. Subsequently, the mixture is activated by heat treatment above the glass transition temperature of the conditioning agent and the temperature is kept constant for about 30 seconds or more, preferably 50 seconds or more, depending on the structure of the stabilised zirconia crystals. Then, the treated zirconia is cooled and remaining conditioning agent is washed.

Acidulated washing solution can be used to wash out remnants of conditioning agent together with the etched zirconia crystals for a time ranging between 30 minutes to 2 hours depending on the surface area requiring treatment. Preferably, this procedure is performed under ultrasonic conditions. If a non-acidulating conditioning agent is used then a more concentrated washing solution is required and the washing time may be doubled. A preferred washing agent in this case is a low concentration acid mixture containing hydrofluoric acid.

After treatment with conditioning agent the surface energy of the treated surface is very high and the surface of the zirconia is very reactive. In addition the structure of zirconia crystals at the surface become well architectured for establishing nano-mechanical retention with many materials. The treated zirconia may be removed from the washing bath and kept in a concentrated alcohol solution. Subsequently a bonding preparation procedure can be performed. Figure 3 shows a SEM image of zirconia after complete treatment with both maturation heat treatments and selective infiltration etching in a combined treatment cycle.

The untreated stabilised zirconia usually has a surface roughness of 0.02 to 0.03 µm when properly polished. This starting surface roughness can be increased by the method of the invention to a value of at least 2 µm for good retention. If required, higher surface roughness can be obtained in thick zirconia samples by prolonging the selective infiltration etching procedure, optionally combined with the maturation heat treatment. This increase in roughness is characterised by the creation of a retentive surface rather than by surface elevations as obtained during sand blasting procedures. A surface roughness of more than 6 µm could be detrimental to the specimen strength. Therefore, preferably the surface roughness lies in the range of 2-6µm.

### Step III: Bonding Preparation Procedure

After the selective infiltration etching, a bonding prepration procedure can be performed.

In one embodiment the bonding preparation procedure comprises the application of a bonding adhesive, which basically comprises a low viscosity epoxy or low viscosity composite resin solution. For the application of the bonding adhesive, other specific chemical agents such as phosphate monomer are not required. Typical examples of such bonding adhesives are for example a mixture of methyl methacrylate monomer or low viscosity Bis-Gma monomer. The zirconia is removed from alcohol and dried. Then, a mixture of preferably non-filled resin is applied and thinned with air. The resin is activated to initiate a polymerisation cycle and after polymerisation the surface is ready for bonding to any available composite material.

In another embodiment, the bonding procedure comprises the application of a liquid mixture of bioactive glass. In particular for medical prostheses or dental implants, a liquid mixture of bioactive glass of choice, for example calcium oxides, phosphosilicate glasses and glass ceramics, may be applied. The bioactive glass may be sintered by heat treatment to form a zirconia surface coated with a thin layer of bioactive material. The coated zirconia is then mildly roughened and sterilised.

In another embodiment, after subjecting the zirconia-based materials to the method of the invention, it can be veneered with a ceramic material of choice. The improved bond strength, from 28MPa to 48MPa, results in improved strength and lifetime of the layered structure.

In yet another embodiment, the bonding preparation procedure comprises the soaking of the treated zirconia in a solution of bioactive glass, human serum or other soft tissue matrix. Hard and soft tissue integrate on the treated surface modified zirconia and thus the zirconia implant becomes accepted and functions within the surrounding tissues.

In accordance with the present invention the bond strength can be improved considerably, from about 25-35 MPa (which are conventional values for *e.g.* a conventional adhesive resin/YPSZ bond) to bond strength values that are much higher *e.g.* up to 60 MPa or more, such as 67 MPa.

The stabilised surface modified zirconia with enhanced bond strength has a wide range of applications, for instance prosthetic restorations, implants, solid electrolytes, fuel cells, oxygen sensors, cutting abrasives, electrical insulators, military fields and many other fields.

### Example 1

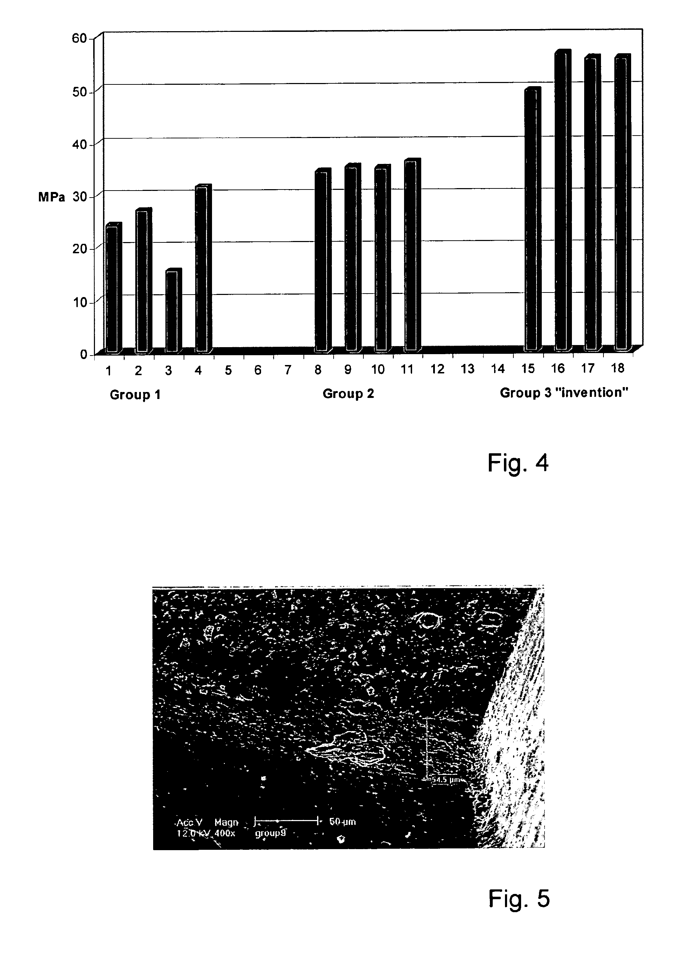

The surface of a 3Y-TZP material subjected to the method of the invention was analysed by SEM, see Figure 3. The zirconia was covered with a resin and then bonded to dental composite material, group 3. The bond strength was determined by a micro tensile bond strength test over four time intervals, immediately after bonding, one day, one week and one month after bonding. Two other commercial systems were used as control, group 1 and 2. Not only did the group subjected to the invention demonstrate more than double the bond values compared to the other commercial system but also the bond strength was stable even under storage of water for one month, see Figure 4.

### Example 2

Stabilised zirconia was subjected to the method of the invention: maturation heat treatment, Selective Infiltration Etching and Bonding Adhesive Procedure. The sample was stored under corrosive chemical solution for two month and later subjected to a Microtensil Bond Strength Test to evaluate the bond strength. A value of 67 MPa was recorded which reflects the stability of the bond under corrosive and active environments. Figure 5 shows a SEM photograph of a zirconia-based material according to the invention that has been bonded to an adhesive resin. Subsequently the sample has been broken. The picture demonstrates that after breaking the prepared sample, adhesive resin (the 54.5 µm layer) still remains on the surface of zirconia. This reflects the superiority of the bond, because prior art zirconia breaks at the borderline of zirconia and adhesive resin.

### Description of Figures.

Figure 1. Normal structure of zirconia showing densely packed zirconia crystals.
Figure 2. Ultra-structural microspacing between zirconia grains after subjecting it to the full heating and cooling cycles as dictated by maturation heat treatment.
Figure 3. Zirconia crystals after final treatment with selective infiltration etching and washing out of the conditioning agent showing less dense surface and inter crystal spacing resulting in highly active and retentive surface.
Figure 4. Bond strength values measured for zirconia subjected to the method of the invention, group 3, compared to comparative systems (group 1 and group 2) over 4 time intervals.
Figure 5. A breaking experiment reflecting a good resin to zirconia bond strength even when stored under corrosive environment.

## Claims

1. Method for increasing the bond strength between stabilised zirconia and another material, wherein said stabilised zirconia comprises ZrO₂ and at least one stabilising compound, which method comprises the successive steps of:
- applying a conditioning agent to said stabilised zirconia;
- heating said stabilised zirconia to a temperature above the glass transition temperature of said conditioning agent;
- cooling said stabilised zirconia; and
- washing said stabilised zirconia.

2. Method according to claim 1, comprising the prior steps of:
i) heating the stabilised zirconia to a heating temperature above 800 °C;
ii) cooling said stabilised zirconia;
iii) optionally repeating step i) and ii);

3. Method according to claim 2, wherein said heating step to a heating temperature above 800 °C is carried out for a period of 5 - 15 minutes, preferably 8 - 12 minutes.

4. Method according to claims 2 or 3, wherein said heating temperature is in the range of 800 - 1100 °C, preferably in the range of 900 - 1000 °C.

5. Method according to any of the preceding claims, which method further comprises the application of a bonding adhesive, a bioactive glass, or mixtures thereof.

6. Method according to any of claims 1-4, which method further comprises soaking of the treated zirconia in a solution of bioactive glass, human serum or other soft tissue matrix.

7. Method according to any of the preceding claims, wherein said stabilised zirconia is yttrium stabilised polycrystalline tetragonal zirconia.

8. Method according to any of the preceding claims, wherein said temperature just above the glass transition temperature of said conditioning agent is maintained for at least 30 seconds, preferably at least 50 seconds.

9. Method according to any of the preceding claims, wherein said conditioning agent is an acidulated or balanced non-ionised oxidised mixture of aluminium, potassium, silica, glass powders and possible trace elements.

10. Surface modified stabilised zirconia, obtainable by a method according to any of the preceding claims.

11. Surface modified stabilised zirconia, which stabilised zirconia comprises ZrO₂ and at least one stabilising compound, which stabilised zirconia has a surface roughness of at least 2 µm.

12. Use of a surface modified stabilised zirconia according to claims 10 or 11 in prosthetic restorations, implants, solid electrolytes, fuel cells, oxygen sensors, cutting abrasives, or electrical insulators.
